# EUROPEAN PATENT SPECIFICATION

(11) **EP 2 820 581 B1**
(45) Date of publication and mention of the grant of the patent: **01.11.2017**
(21) Application number: 13718895.9
(22) Date of filing: 26.02.2013
(51) Int. Cl.: G06F 19/22, C12Q 1/68

(54) **COMPACT NEXT GENERATION SEQUENCING DATASET AND EFFICIENT SEQUENCE PROCESSING USING SAME**
KOMPAKTER SEQUENZIERUNGSDATENSATZ DER NÄCHSTEN GENERATION UND EFFIZIENTE SEQUENZVERARBEITUNG DAMIT
ENSEMBLE DE DONNÉES DE SÉQUENÇAGE DE PROCHAINE GÉNÉRATION COMPACT ET TRAITEMENT DE SÉQUENCE EFFICACE L'UTILISANT

(30) Priority: 28.02.2012 US 201261604014 P
(43) Date of publication of application: 07.01.2015
(73) Proprietor: Koninklijke Philips N.V., 5656 AE Eindhoven (NL)
(72) Inventor: KUMAR, Subodh, NL-5656 AE Eindhoven (NL); SINGH, Randeep, NL-5656 AE Eindhoven (NL); CHAKRABARTI, Biswaroop, NL-5656 AE Eindhoven (NL); KUMAR, Sunil, NL-5656 AE Eindhoven (NL)
(74) Representative: Steffen, Thomas
(86) International application number: PCT/IB2013/051531
(87) International publication number: WO 2013/128371

(56) References cited:
- EP-A2- 2 634 717
- PATEL R. K. ET AL: "NGS QC Toolkit: A Toolkit for Quality Control of Next Generation Sequencing Data", PLOS ONE, vol. 7, no. 2, 1 February 2012 (2012-02-01), page e30619, XP055088926, ISSN: 1932-6203, DOI: 10.1371/journal.pone.0030619
- KIRCHER M. ET AL: "Improved base calling for the Illumina Genome Analyzer using machine learning strategies", GENOME BIOLOGY, BIOMED CENTRAL LTD., LONDON, GB, vol. 10, no. 8, 14 August 2009 (2009-08-14), page R83, XP021061445, ISSN: 1465-6906, DOI: 10.1186/GB-2009-10-8-R83
- SCHADT E. E. ET AL: "A window into third-generation sequencing", HUMAN MOLECULAR GENETICS, vol. 19, no. R2, 15 October 2010 (2010-10-15), pages R227-R240, XP055036493, ISSN: 0964-6906, DOI: 10.1093/hmg/ddq416
- MCKENNA A. ET AL: "The Genome Analysis Toolkit: A MapReduce framework for analyzing next-generation DNA sequencing data", GENOME RESEARCH, vol. 20, no. 9, 19 July 2010 (2010-07-19), pages 1297-1303, XP055089173, ISSN: 1088-9051, DOI: 10.1101/gr.107524.110

## Description

The following relates to the genetic analysis arts, and to applications of same such as the medical arts including oncology arts, veterinary arts, and so forth.

Efficient genetic sequencing systems, sometimes referred to as "next generation sequencing" (NGS) systems, are capable of sequencing an entire genome rapidly and substantially automatically. Existing NGS systems sometimes exhibit lower read accuracy as compared with slower conventional techniques such as Sanger sequencing, although NGS accuracy is already sufficient for clinical applications and is expected to improve as the technologies mature.

To assess read accuracy (or reliability), a base quality score is typically computed for each base of a read. In the case of Sanger sequencing, phred quality scores are calculated from spectrogram data by calculating parameters for the sequenced base such as peak shape and resolution, and comparing these values with an empirically developed look-up table. The phred scores are generally considered to be logarithmically related to the probability that the base was called incorrectly. For example, a phred score (Q) of Q=20 corresponds to a 99% base call accuracy, while Q=30 corresponds to 99.9% accuracy, Q=40 corresponds to 99.99% accuracy, and so forth. NGS approaches employ parallel processing techniques that enhance throughput by orders of magnitude.

The conventional phred score calculation is not strictly applicable to NGS techniques, but most NGS platforms generate "phred-like" base quality scores that are comparable to or scaled to conventional phred scores computed from spectrogram data. In the art, these "phred-like" base quality scores are sometimes referred to as phred scores.

A common storage format for the reads is the FASTA format, in which occurrences of the bases adenine (A), cytosine (C), guanine (G), and thymine (T) are represented by ASCII letters "A", "C", "G", and "T", respectively. For RNA the base thymine (T) is replaced by the base uracil (U). FASTA does not store quality score information.

On the other hand, the FASTQ format incorporates quality scores. A FASTQ representation of a read is typically in ASCII format and includes four lines: a header line as the sequence identifier; a sequence line listing the ordered sequence of bases represented by the appropriate ASCII letters "A", "C", "G", and "T" (for DNA) or "U" (for RNA); a separator line typically consisting solely of a plus ("+") sign (in some FASTQ formats this line may also include other information such as the sequence identifier, e.g. mirroring the header); and a base quality scores line listing the base quality scores using suitable encoding (for example, mapping phred scores 0-93 to ASCII codes 33-126 so as to avoid numerous "unprintable" ASCII characters in the 0-32 range).

The total storage used for a FASTQ read entry is therefore 2L_{seq}+[H]+K, where L_{seq} is the read length (i.e., the number of bases making up the read) and [H] is the number of characters in the header. The "+K" is a constant offset that accounts for the "+" separator and for any delimiter characters (e.g., carriage return or carriage return/line feed) separating the four lines, while the factor of 2 multiplier accounts for the fact that each base is represented by both a base character ("A", "C", "G", "T", or "U") and a base quality score. For reads of reasonable length (e.g., more than a few bases) the bulk of the entry storage consists of the sequence and the total storage is approximately 2L_{seq}.

The size of the raw reads file can be large in NGS. For a whole genome dataset, a minimum size is imposed by the number of bases in the genome, e.g. of order 3 billion base pairs (bps) in a complete haploid human genome. In practice, the raw reads file is substantially larger than this due to overlaps and duplications amongst the reads, as well as the storage of the base quality values. A whole genome dataset for a human subject can therefore be of order several gigabytes. As an illustrative example, each genome sequenced with 100 bp reads and 30x coverage may consist of a total of ∼150 GB data divided into multiple files. A typical FASTQ file is of size ∼6.9GB, about 20 such files may be sufficient to store a whole human genome. A single file of size ∼6.9 GB can be reduced to about 2.3 GB by storing in a compressed gzip format.

Patel et al. (PLos One 2012: 7(2) e30619) describe quality control techniques in a tool that could be used on sequence data generated using next generation sequence technologies. More particularly, tools that would filter reads shorter than given length cut-off at several steps, trim reads containing certain sequences and filter filtered sequence reads based on a Phred quality score are described.

The combination of large genome dataset size and rapidly decreasing cost of performing NGS means that genetic data storage is a substantial portion of the total cost of sequencing applications, and moreover this portion of the cost is expected to continue to increase as the sequencing becomes less costly and produces larger data sets. Moreover, the large raw reads dataset translates into higher computational cost for downstream processing such as alignment.

The following contemplates improved apparatuses and methods that overcome the aforementioned limitations and others.

According to one aspect, an apparatus according to independent claim 1 is provided. According to another aspect, a method according to independent claim 9 is provided. According to another aspect, a non-transitory storage medium according to independent claim 13 is provided. One advantage resides in reduced storage for reads without a corresponding loss of significant informational content.

Another advantage resides in faster and more efficient alignment.

Another advantage resides in facilitating efficient statistical characterization of overall read quality.

Another advantage resides in providing the foregoing advantages while retaining a text format for the reads storage and while retaining compatibility with existing downstream processing components such as aligners.

Numerous additional advantages and benefits will become apparent to those of ordinary skill in the art upon reading the following detailed description.

The invention may take form in various components and arrangements of components, and in various process operations and arrangements of process operations. The drawings are only for the purpose of illustrating preferred embodiments and are not to be construed as limiting the invention.
FIGURE 1 diagrammatically shows a genetic analysis system including compact storage of reads as described herein.
FIGURE 2 shows a portion of a raw reads file in conventional FASTQ format.
FIGURE 3 diagrammatically shows operation of the reads formatting module of the system of FIGURE 1.
FIGURE 4 shows the raw reads file portion of FIGURE 2 after processing by the reads formatting module of the system of FIGURES 1 and 3.
FIGURE 5 shows the raw reads file portion of FIGURE 4 after processing by the optional reads reformatting module of the system of FIGURE 1.

Disclosed herein are approaches for formatting raw read data including base quality scores in a way that retains most of the useful information while allowing for substantial reduction in file size. As discussed previously, in the conventional FASTQ format a read occupies somewhat more than 2L_{seq} (ASCII) characters where L_{seq} is the number of bases. Other existing text-based storage formats that store both the base sequence and corresponding base quality scores occupy a comparable amount of storage. For example, in the Qseq format both the base sequence and the quality scores are stored, but arranged in a single line of text. The FASTA format can reduce this storage by roughly half - but it does so by losing all base quality scoring information. Alternatively, one can convert the text formatted read entry to a non-text format (e.g., a binary format in which two bits encode the base and the phred score is represented by a binary integer value). However, most downstream processing components (e.g., aligners, variant annotators, and so forth) are designed to process reads in text format. The disclosed approaches retain all of the base sequence information and retain substantial useful base quality information, in text format, while still allowing substantial reduction in the read storage size.

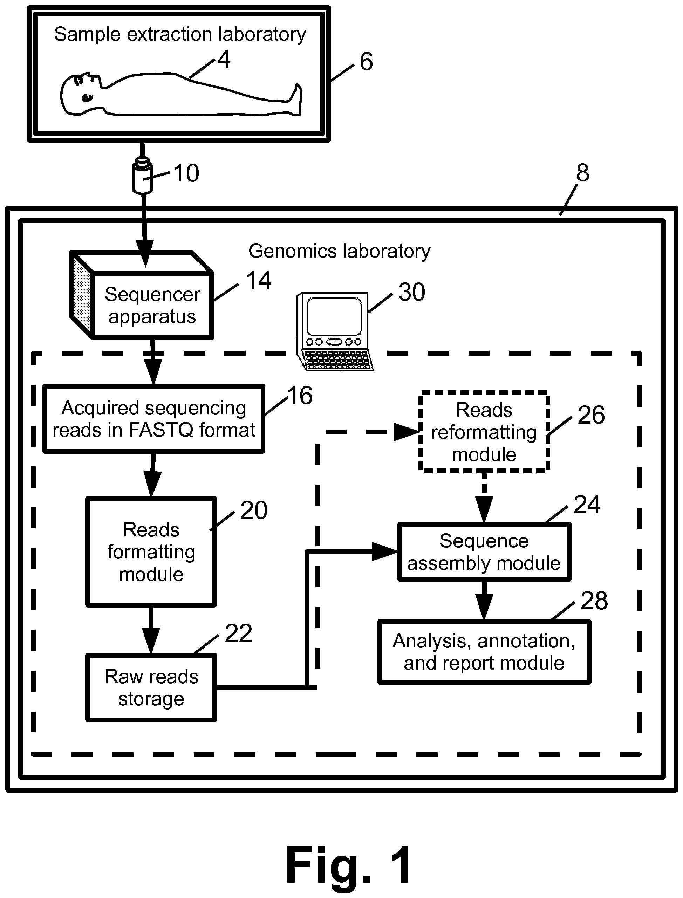

With reference to FIGURE 1, an illustrative clinical or diagnostic application is described. A patient **4** undergoes tissue sample extraction in a sample extraction laboratory **6** to generate a tissue sample that is processed by a genomics laboratory **8** to sequence and analyze DNA and/or RNA of the sample. For example, the sampling laboratory **6** may extract from the subject **4** a tissue sample from a malignant lesion, optionally along with a normal tissue sample extracted from elsewhere in the patient **4**. As some other illustrative examples, the sample extraction may comprise a biopsy procedure employing a biopsy needle or other interventional instrument, the plucking of a hair sample to obtain the follicle containing DNA, the drawing of a blood using a hypodermic needle, or so forth. FIGURE 1 shows an illustrative extracted tissue sample **10**. It is noted that while the illustrative embodiment is operative on patient **4** in a clinical application, in other applications the tissue sample may be extracted from a nonhuman subject such as a veterinary subject, a biology research subject, or so forth. The application of the genetic analysis can be varied, such as: medical or veterinary diagnosis, monitoring, or the like; generating a genetic database for anthropology studies (e.g., population migration studies, et cetera); subject assessment in a clinical or preclinical study; or so forth. It will also be noted that in illustrative FIGURE 1 the sample **10** is represented by an illustrative vial; however, it is to be understood that the sample **10** may in general take any form suitable for the type of tissue that has been sampled, and may be contained or supported by any suitable container or support for that type of tissue. For example, the sample **10** may be a fluid sample, a surface sample (e.g. obtained by oral swabs and disposed on a sterile slide or other suitable surface), or so forth.

At the genomics laboratory **8**, the tissue sample **10** is processed by a sequencer apparatus **14** to generate sequencing reads. The sequencer apparatus **14** may be a next generation sequencing (NGS) apparatus or a more conventional sequencing apparatus such as a Sanger sequencing facility. The sequencer apparatus **14** may in some embodiments be a commercial sequencing apparatus such as are available from Illumina, San Diego, CA, USA; Knome, Cambridge, MA, USA; Ion Torrent Inc., Guilford, CT, USA; or other NGS system vendors; however, a noncommercial or custom-built sequencer is also contemplated. A portion of the functionality of the sequencer apparatus may also be performed manually. The generated sequencing reads are optionally filtered to remove duplicate reads and/or to discard reads having base quality (e.g. phred) scores below 20 (or below another selected base quality score threshold). The remaining sequencing reads **16** are suitably formulated in a FASTQ format (as shown) or in another format that includes a base quality value for each base of the read (e.g., Qseq). In embodiments in which the sequencer apparatus **14** is an automated commercial product, the sequencer apparatus **14** is suitably operated in its usual and ordinary manner and outputs the sequencing reads **16** formatted in a standard output of the sequencer apparatus **14**.

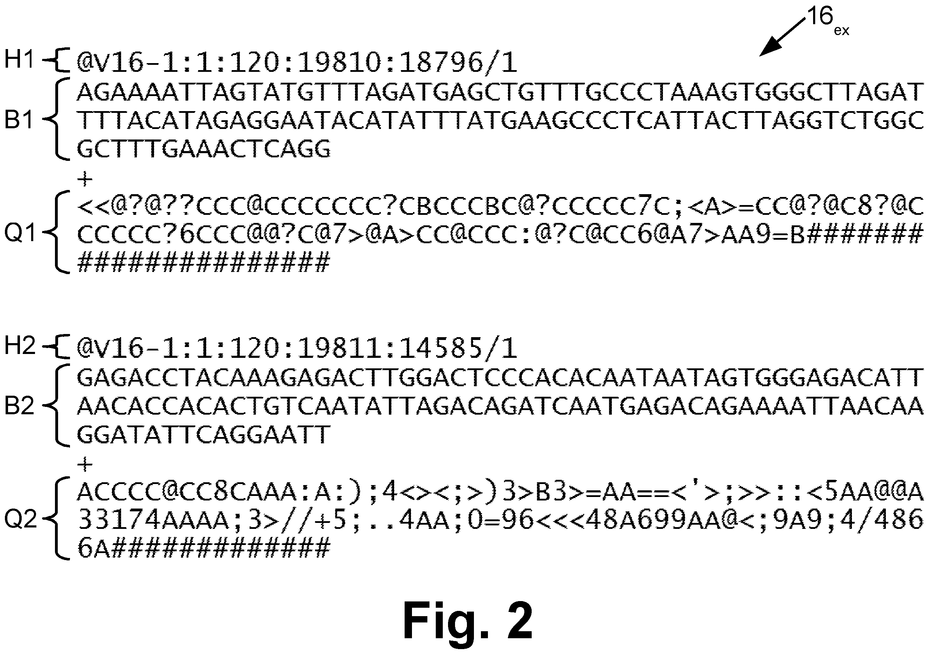

With continuing reference to FIGURE 1 and with further reference to FIGURE 2, in the illustrative examples the sequencing reads **16** are assumed to be in a FASTQ format. FIGURE 2 shows two reads **16ₑₓ.** The first sequencing read includes a header **H1,** a base sequence **B1** for the first read, a separator line containing only a plus sign (+), and a base quality scores sequence **Q1** having base quality scores represented as letters corresponding to the bases of the first read sequence **B1.** The second sequencing read includes a header **H2**, a base sequence **B2** for the second read, a separator line containing only a plus sign (+), and a base quality scores sequence **Q2** having base quality scores represented as letters corresponding to the bases of the second read sequence **B2**. More generally, each sequencing read is represented in FASTQ format as text and includes: (1) a base sequence defining the read (e.g., the base sequence **B1** for the first read and the base sequence **B2** for the second read) and (2) a sequence of base quality scores (e.g., the base quality scores **Q1, Q2** for the first and second sequencing reads, respectively) having a base quality score for each base of the base sequence. In other text formats these components (1, 2) may be organized differently - for example, in Qseq the base sequence (1) and corresponding base quality scores (2) form a single line of text, rather than two separate lines as in the FASTQ format. Moreover, the auxiliary elements of the sequencing read representation (such as the headers **H1**, **H2** and separator plus signs in the example of FIGURE 2) can have various formats, may be omitted entirely, or alternatively auxiliary elements other than those shown in FIGURE 2 may be included in the read representation. Still further, while the illustrative textual read representations are in ASCII text, other text encodings are contemplated such as an "extended" ASCII encoding (in which values in the range [128-256] decimal are used to encode "special" characters).

The illustrative reads **16ₑₓ** of FIGURE 2 are DNA reads, as is recognizable from the inclusion of some thymine (T) bases. Alternatively, the reads may be RNA reads. RNA does not include thymine bases, but instead may include uracil (U) bases. The base quality scores **Q1, Q2** employ a conventional phred quality score format in which the quality score lies in the interval [0,93] inclusive and is encoded into ASCII by adding an offset of 33 so that the ASCII codes for the characters of lines **Q1, Q2** lie in the range [33,126] inclusive. This offset ensures that most or all of the characters are printable characters. (However, some display devices and/or printers may be unable to print certain codes, such as the grave accent symbol typically corresponding to ASCII code 96 decimal). These are merely illustrative examples, and other base quality score scales and/or text encoding schemes can be used.

With continuing reference to FIGURE 1, a reads formatting module **20** receives the reads **16** in the FASTQ format (or more generally receives a sequencing read comprising at least a base sequence and a corresponding set of base quality scores). The reads formatting module **20** performs formatting of the reads including at least replacement of the sequences of base quality scores **Q1, Q2** by text indicative of base sub-sequences whose base quality scores satisfy a specified threshold. The effect of this is to substantially reduce the size of the textual read representation while retaining the most relevant information provided by the base quality scores.

The disclosed operation of the reads formatting module **20** is based on the following observations that are made herein. For many applications, such as deciding the offset for use in alignment, what is important is defining a sub-sequence of "good" bases, that is, bases whose quality scores are above a certain base quality score threshold. What is usually not done is to attempt to mine one, or a few, "good" bases that are interspersed amongst a high density of surrounding "bad" bases. Such a "base mining" approach would slow down the processing; additionally, a mined "good" base that is surrounded by "bad" neighboring bases would be suspect despite its high base quality score since the region as a whole is of questionable quality.

Thus, the relevant information provided by the base quality scores text **Q1**, **Q2** is usually not the precise quality score for each individual base. Rather, the relevant information is the identity of the principal contiguous sub-sequence of "good" bases in the read. A "good" base is suitably quantified as a base whose quality score satisfies a specified base quality score threshold. By "satisfying" the threshold, it is meant that the quality score equals or exceeds the threshold, e.g. quality scores of 40 and higher satisfy a threshold of 40; or, in an equivalent alternative formulation employing a strict inequality, the threshold can be set to 39 and the threshold is "satisfied" by quality scores that strictly exceed this threshold (again, these are quality scores of 40 and higher assuming integer values for the quality scores). As used herein, the phrase "satisfy a threshold" and similar phraseology encompasses embodiments employing a threshold operating in conjunction with an "equal to or greater than" inequality and also embodiments employing a threshold operating in conjunction with a strictly "greater than" inequality. In view of this, the reads formatting module **20** identifies the longest subsequence of bases for which all the bases have base quality scores above a base quality threshold value. The precise base quality scores for the bases are lost, but the downstream instrument or processing is assured that all bases in the identified longest sub-sequence have quality scores greater than the threshold value. This may optionally be done for a single threshold, or for two, three, or more different thresholds. The optional use of multiple thresholds enables flexibility in deciding how "good" the bases should be - that is, a downstream instrument or process can select between a shorter sequence of higher assured base quality (by selecting the sub-sequence defined by the highest threshold) or a longer sequence of lower assured base quality (by selecting the sub-sequence defined by the lowest threshold). This is very useful for alignment algorithms that are hash based, as the contiguous quality bases can be used as a key for aligning the read to a reference.

With continuing reference to FIGURE 1, the reads in the more compact format output by the reads formatting module **20** are stored in raw reads storage **22**. The stored sequencing reads are subsequently processed by one or more downstream instruments or processes. For example, in illustrative FIGURE 1 the raw reads are assembled by a sequence assembly module **24** to generate a genetic sequence for the tissue sample. The assembly performed by the assembly module **24** can be *de novo* alignment of overlapping portions sequencing reads, or can be mapping of the sequencing reads to a reference sequence while allowing for a certain fraction (e.g., 5-10%) of base mismatches. In the latter case the reference sequence can be, for example, a standard reference sequence of the human genome in the illustrative case of human patient **4**. The alignment processing includes defining offsets for reads to account for non-intrinsic effects (e.g., contaminants, type of sequencing chemistry, or so forth which tends to corrupt the ends of the read). The base quality scores are typically consulted in determining these offsets. The assembly module **24** is suitably modified to use the modified base quality information output by the reads formatting module **20**. The resulting aligned sequence dataset can be variously stored, compared with other sequences, analyzed to detect probative variants, or so forth. In the illustrative example, an analysis, annotation, and report module **28** compares the aligned sequence with an annotated reference sequence in order to identify variants that correlate with a disease (e.g., a type of cancer) or other information.

In an alternative embodiment, the assembly module **24** (or other downstream instrument or process) is a conventional component of a type designed to receive the raw reads in the FASTQ format. In this alternative embodiment, there is no modification made to the assembly module **24**. Instead, a front-end component **26** is provided which reformats the compact reads stored in the storage **22** to a format comporting with FASTQ. This entails labeling all bases within the identified sub-sequence of "good" bases with "good" base quality scores (that is, with base quality scores above the base quality score threshold) and formatting these labels using the conventional FASTQ format. The expansion does not completely replicate the original (e.g., FASTQ) base quality scores sequence, but provides substantially equivalent base quality information represented in the FASTQ format.

In the approach of FIGURE 1, the sequencer apparatus **14** outputs the sequencing reads **16** in conventional FASTQ format, which is then converted to the more compact format disclosed herein by the reads formatting module **20** before storage of the reads. This is advantageous for retrofitting an existing sequencer apparatus **14** to employ the disclosed more compact format. However, it is also contemplated to integrate the sequencer apparatus **14** and the reads formatting module **20** as a single unit that performs the sequencing and outputs the reads in the disclosed more compact format. In such embodiments there is no generation of the intervening FASTQ-formatted data **16**.

The various processing components **20**, **24**, **26**, **28** are suitably embodied by an illustrative computer or other electronic data processing device **30**. By way of illustration, the electronic data processing device **30** may include: a notebook computer; a desktop computer; a mobile device such as a smartphone, tablet computer, personal data assistant (PDA), or the like; a network server computer accessible via the Internet and/or a local wired/wireless data network; various combinations thereof; or so forth. The raw reads storage **22** is suitably embodied as random access memory (RAM), flash memory, or other type of electronic storage, or as a hard disk or other type of magnetic storage, or so forth, wherein the electronic, magnetic, or other type of storage is configured to store the raw reads in the compact text format output by the reads formatting module **20** for later retrieval by an electronic data processing device.

The disclosed techniques for processing raw reads in memory-efficient and computationally-efficient ways are also suitably embodied as a non-transitory storage medium storing instructions executable by the illustrative computer or other electronic data processing device **30** to perform the disclosed reads processing. The non-transitory storage medium storing the executable instructions may, for example, include: a hard disk drive or other magnetic storage medium; an optical disk or other optical storage medium; a flash memory, random access memory (RAM), read-only memory (ROM), or other electronic storage medium; or so forth.

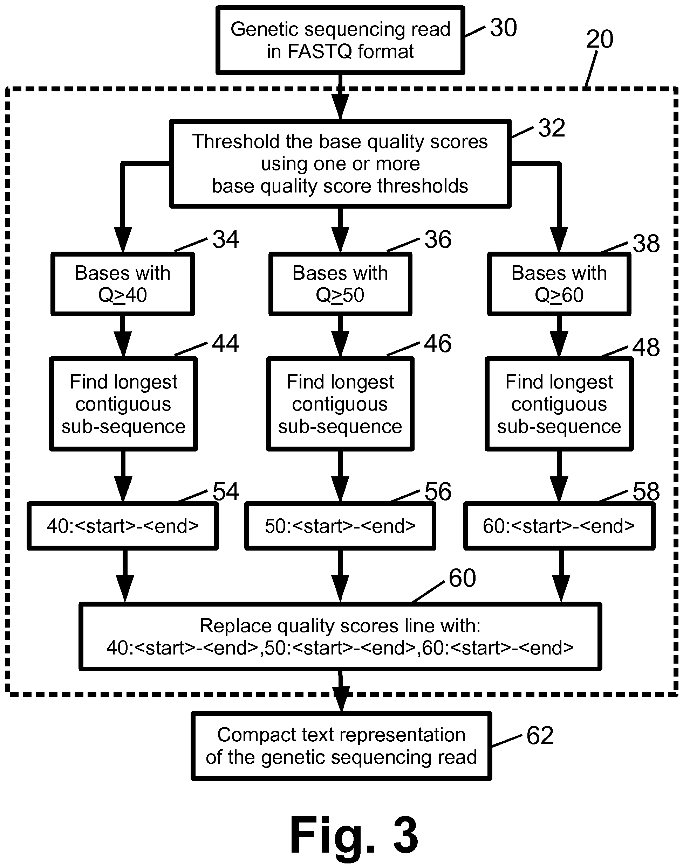

With continuing reference to FIGURE 1 and with further reference to FIGURE 3, illustrative operation of the reads formatting module **20** is described for a genetic sequencing read **30** in FASTQ format. More generally, the input to the reads formatting module **20** is a genetic sequencing read **30** comprising an ordered sequence of nucleotide bases (also referred to herein as a base sequence) having a corresponding ordered sequence of base quality scores. In an operation **32**, the base quality scores are thresholded using one or more base quality score thresholds. In the illustrative examples, a base quality score is represented by the symbol Q and lies in the range three base quality score thresholds are used, namely thresholds of 40, 50, and 60 in the illustrative example. More generally, the thresholds can be of different levels than these, and the total number of thresholds can be one, two, three (as in the example), four, or more. The thresholds are optionally user defined thresholds, although hard-coded and/or automatically generated threshold values are also contemplated. The threshold or thresholds are preferably generally accepted by a research community or group as defining bases of low, medium and high quality, (or bronze, silver, gold, platinum, or some other generally accepted quality scale). In general, the threshold or thresholds can be chosen based on an individual researcher's preference, or may be an output of a pre-processing step on raw reads that defines these thresholds based on raw reads characteristics. The thresholds may be fixed and pre-decided for a particular sequencing instrument as well. Thus, the output of the thresholding operation **32** is the set of bases having Q≥40 **34**, the set of bases having Q≥50 **36**, and the set of bases having Q≥60 **38**. (An equivalent alternative employing a strict inequality is to employ thresholds 39, 49, and 59 which are satisfied by bases having Q>39, Q>49, and Q>59, respectively.) Using the information **34**, the longest contiguous sub-sequence of bases having Q≥40 is identified in operation **44**. Using the information **36**, the longest contiguous sub-sequence of bases having Q≥50 is identified in operation **46**. Using the information **38**, the longest contiguous sub-sequence of bases having Q≥60 is identified in operation **48**. The longest contiguous sub-sequence of bases having Q≥40 is suitably represented by the text string "40: <start>-<end>" **54** (also referred to herein as the base quality text field) where <start> denotes the text representation of the position of the first nucleotide base of the longest sub-sequence with Q≥40 in the ordered sequence of nucleotide bases and <end> denotes the text representation of the position of the last nucleotide base of the longest sub-sequence with Q≥40 in the ordered sequence of nucleotide bases. Similarly, the longest contiguous sub-sequence of bases having Q≥50 is suitably represented by the base quality text field "50: <start>-<end>" **56**, and the longest contiguous sub-sequence of bases having Q≥60 is suitably represented by the base quality text field "60: <start>-<end>" **58**. In an operation **60**, the FASTQ base quality score sequence is replaced by a concatenation of the base quality text fields **54**, **56**, **58**, that is, the FASTQ base quality score sequence is replaced by the text string: "40: <start>-<end>, 50: <start>-<end>, 60: <start>-<end>". The final output is a compact text representation **62** of the genetic sequencing read which includes: (1) the text string representing the ordered sequence of nucleotide bases (suitably carried over unmodified from the FASTQ format, although some reformatting is optionally contemplated) and (2) one or more base quality text field wherein each base quality text field identifies the longest sub-sequence of the ordered sequence of nucleotide bases for which the corresponding base quality scores exceed the base quality score threshold.

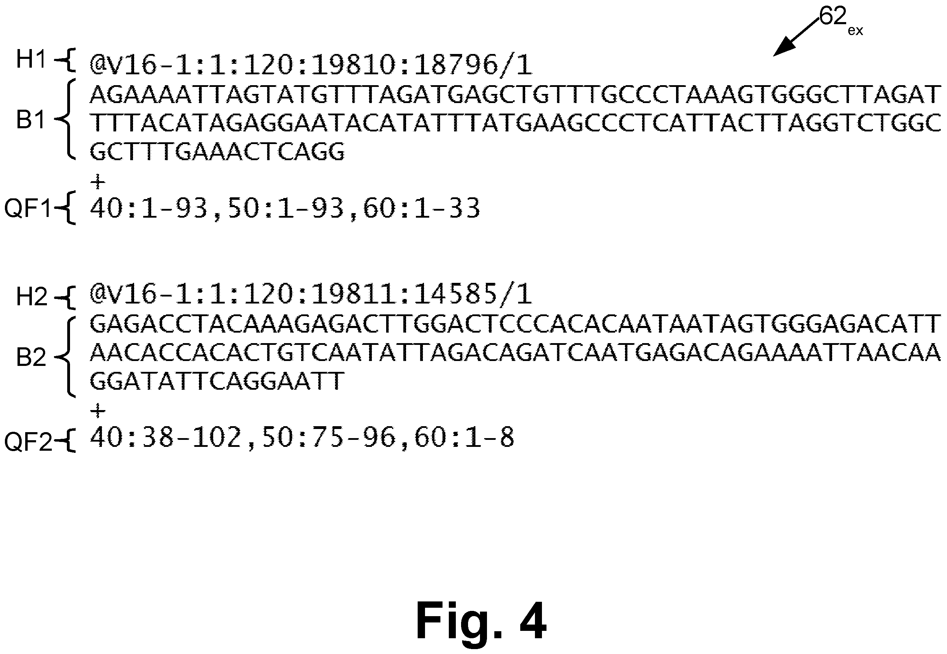

With reference back to FIGURE 2 and with further reference to FIGURE 4, the reads **16ₑₓ** in FASTQ format shown in FIGURE 2 are shown in compact text representation **62ₑₓ** in FIGURE 4. The headings **H1, H2** and base sequences **B1, B2** are carried over from the FASTQ representation without modification. However, the corresponding sequences of base quality scores **Q1, Q2** are replaced by base quality text fields **QF1**, **QF2** having the format output by the concatenation operation **60** (see FIGURE 3).

Some observations may be made regarding the base quality text fields disclosed herein. First, the longest sub-sequence of bases having base quality scores exceeding a given base quality score threshold is identified - accordingly, there is only one sub-sequence per base quality score threshold. Second, the length of the sub-sequence for any lower base quality score threshold will be longer than or equal to the length of the sub-sequence for any higher base quality score threshold.

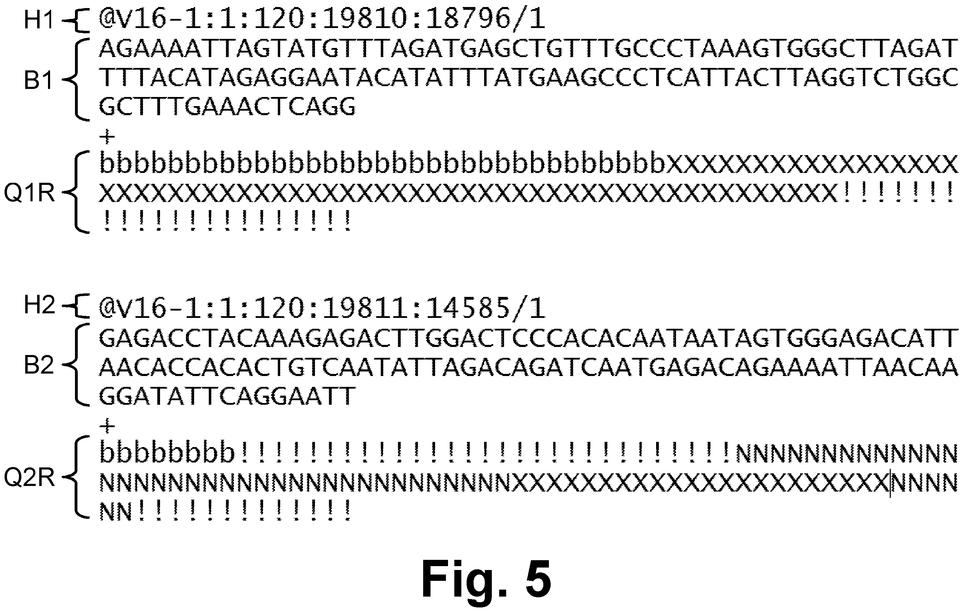

With reference to FIGURE 5, operation of the optional reads reformatting module **26** shown in FIGURE 1 is described, using the example of FIGURE 4 as the input compact text representation of the sequencing read. The reads reformatting module **26** is suitably employed if a downstream instrument or process requires a conventional sequencing reads format such as a FASTQ formatted read. The example of FIGURE 5 is the reformatted version of the compact text representation **62ₑₓ** of FIGURE 4. The headings **H1**, **H2** and the base sequences **B1**, **B2** are not affected by the reformatting. However, the base quality text fields **QF1**, **QF2** are each converted to an ordered sequence of reconstituted base quality scores **Q1R**, **Q2R** corresponding to the ordered sequence of nucleotide bases **B1**, **B2**. The reconstituted base quality scores for bases of the longest sub-sequence identified by the base quality text field are set to be greater or equal to base quality threshold. In the illustrative example, the reconstituted base quality scores for the bases in the longest sub-sequence with Q ≥ 60 (namely the sub-sequence [1,33] for the first read and the sub-sequence [1,8] for the second read) are set to the value 65 (corresponding to ASCII code 98, i.e. lowercase "b", using the offset +33 as in the FASTQ data of FIGURE 2 where phred scores 0-93 are mapped to ASCII codes 33-126). The reconstituted base quality scores for the bases in the longest sub-sequence with Q ≥ 50 are set to the value 55 (corresponding to ASCII code 88, i.e. uppercase "X"). For the first sequencing read, the sub-sequence set to "X" is [34,93]. (Note that the overlapping sub-sequence [1,33] which is part of both the longest sub-sequence with Q ≥ 50 and the longest sub-sequence with Q ≥ 60 is set to the higher reconstituted value, i.e. Q=65, which greater than both thresholds 50 and 60.) For the second read the sub-sequence [75,96] is set to "X". The reconstituted base quality scores for the bases in the longest sub-sequence with Q ≥ 40 are set to the value 45 (corresponding to ASCII code 78, i.e. uppercase "N"). For the first sequencing read, this sub-sequence is coextensive with the sub-sequence for Q ≥ 50, and so no bases are assigned reconstituted quality score=45 (ASCII "N"). For the second read the sub-sequences [38,74] and [97,102] are set to "N"; here again, the overlapping sub-sequence [75,96] is assigned reconstituted quality score=55 (ASCII "X"). Finally, any bases that are not part of any of the longest-sub-sequences having base quality scores greater than a given base quality score threshold are assigned a low base quality score, namely Q=0 (ASCII code 33 corresponding to the character "!").

Comparing FIGURES 2 and 5, the resulting reconstituted base quality score sequences **Q1R, Q2R** are seen to be different from the original base quality score sequences **Q1, Q2** generated by the sequencing. However, the reconstituted base quality score sequences **Q1R, Q2R** retain the salient information, namely the longest sub-sequence with Q≥40, Q≥50, and Q≥60 is retained. Thus, an application such as the illustrative sequence assembly **24** (see FIGURE 1) will correctly identify the longest sub-sequence of "good" bases, as defined by the chosen base quality score threshold.

On the other hand, if the sequence assembly **24** is modified to use the base quality text fields **QF1**, **QF2** of FIGURE 4 then the alignment processing can be simplified. During the alignment process, for example, using a Burrows-Wheeler Aligner (BWA), an offset of a few bases at the beginning and end of each read is assigned. Generally a BWA offset defined as 10-15% of the read length is employed to nullify extraneous effects (e.g., type of sequencing chemistry, contaminations et cetera), but this is merely a rough estimate and may not accurately reflect the actual extraneous ends of the read. When using the base quality text fields disclosed herein, the alignment process can suitably define an offset boundary for the genetic sequencing read based on the content of the base quality text field. For example, the offset boundary can be defined as a boundary of the longest sub-sequence identified for a chosen base quality score threshold. More generally, any downstream instrument or process that heretofore has utilized base quality scores has typically needed to perform complex processing to analyze the FASTQ base quality scores sequence in order to identify contiguous regions of "good" bases. Here, this processing is performed *a priori* and is stored as part of the compact text representations of the raw reads, thus enabling more efficient downstream processing.

In the illustrative examples, the base quality score threshold is stored as part of the base quality text field. However, if the threshold is a fixed value (for example, if the illustrative three thresholds 40, 50, 60 are always used) then the base quality text field optionally omits storing the base quality score threshold, and instead stores only the identification of the longest sub-sequence of the ordered sequence of nucleotide bases for which the corresponding base quality scores exceed the base quality score threshold.

In the illustrative examples, the base quality text field stores the identification of the longest sub-sequence for which the base quality scores exceed the threshold by storing the positions of the first and last nucleotide bases of the sub-sequence in the ordered sequence of nucleotide bases. However, other formats can be used. For example, the position of the first nucleotide base of the sub-sequence in the ordered sequence of nucleotide bases and the number of nucleotide bases in the sub-sequence can be stored, thus providing equivalent information.

## Claims

1. An apparatus comprising:
an electronic data processing device **(30)** configured to generate a compact text representation of a genetic sequencing read, the genetic sequencing read comprising an ordered sequence of nucleotide bases having a corresponding ordered sequence of base quality scores, the compact text representation including (1) a text string representing the ordered sequence of nucleotide bases and (2) a base quality text field indicative of the longest sub-sequence of the ordered sequence of nucleotide bases for which the corresponding base quality scores satisfy a base quality score threshold, wherein the electronic data processing device identifies the longest sub-sequence of the ordered sequence of nucleotide bases; and
a raw reads storage **(22)** configured to store the compact text representation.

2. The apparatus of claim 1, wherein the compact text representation of the genetic sequencing read includes:
(2.1) a first base quality text field indicative of the longest sub-sequence of the ordered sequence of nucleotide bases for which the corresponding base quality scores satisfy a first base quality score threshold, and
(2.2) a second base quality text field indicative of the longest sub-sequence of the ordered sequence of nucleotide bases for which the corresponding base quality scores satisfy a second base quality score threshold that is higher than the first base quality score threshold.

3. The apparatus of any one of claims **1-2**, wherein the compact text representation of the genetic sequencing read does not include a text string representing the ordered sequence of base quality scores.

4. The apparatus of any one of claims **1-3,** wherein the base quality text field indicates the longest sub-sequence by specifying one of:
the position of the first nucleotide base of the sub-sequence in the ordered sequence of nucleotide bases and the position of the last nucleotide base of the sub-sequence in the ordered sequence of nucleotide bases; and
the position of the first nucleotide base of the sub-sequence in the ordered sequence of nucleotide bases and the number of nucleotide bases in the sub-sequence.

5. The apparatus of any one of claims **1-4**, wherein the base quality text field further indicates the base quality score threshold.

6. The apparatus of any one of claims **1-5**, wherein the electronic data processing device **(30)** is further configured to align genetic sequencing reads including said genetic sequencing read to reconstruct a genetic sequence, wherein the alignment process includes defining an offset boundary for said genetic sequencing read based on the content of the base quality text field.

7. The apparatus of claim 6, wherein the offset boundary is defined as a boundary of the longest sub-sequence identified in the base quality text field.

8. The apparatus of any one of claims **1-7**, wherein the electronic data processing device **(30)** is further configured to expand the compact text representation of the genetic sequencing read to generate an expanded text representation of the genetic sequencing read including (1') a text string representing the ordered sequence of nucleotide bases and (2') an ordered sequence of reconstituted base quality scores corresponding to the ordered sequence of nucleotide bases;
wherein the reconstituted base quality scores are greater than the base quality threshold for bases of the longest sub-sequence identified by the base quality text field.

9. A method operative on a genetic sequencing read comprising a base sequence acquired by processing a tissue sample **(10)**, the method comprising:
generating a compact text representation of the genetic sequencing read, the genetic sequencing read comprising an ordered sequence of nucleotide bases having a corresponding ordered sequence of base quality scores, the compact text representation including (1) a text string representing the base sequence and (2) a base quality text field indicative of the longest sub-sequence of the base sequence for which base quality scores of the bases of the sub-sequence satisfy a base quality score threshold; and
storing the compact text representation of the genetic sequencing read in a raw reads storage **(22)**;
wherein the generating is performed by an electronic data processing device **(30)**, and wherein the electronic data processing device identifies the longest sub-sequence of the ordered sequence of nucleotide bases.

10. The method of claim **9**, wherein:
the compact text representation of the genetic sequencing read does not include a text string representing a sequence of base quality scores corresponding to the base sequence; and
the text string representing the base sequence comprises a sequence of letters comporting with a base representation code in which the letter "A" or "a" represents an adenine base, the letter "C" or "c" represents a cytosine base, the letter "G" or "g" represents a guanine base, the letter "T" or "t" represents a thymine base, and the letter "U" or "u" represents a uracil base.

11. The method of any one of claims **9-10**, wherein:
the base quality text field indicates the longest sub-sequence by specifying one of: (i) the first base position and last base position of the sub-sequence and (ii) the first base position and the number of bases in the sub-sequence; and
the base quality text field further identifies the base quality score threshold.

12. The method of any one of claims **9-11**, further comprising:
aligning genetic sequencing reads including said genetic sequencing read to reconstruct a genetic sequence wherein the aligning includes defining an offset boundary for said genetic sequencing read as a boundary of the longest sub-sequence identified in the base quality text field;
wherein the aligning is performed by an electronic data processing device **(30).**

13. A non-transitory storage medium storing instructions executable by an electronic data processing device (30) to process a genetic sequencing read, the genetic sequencing read comprising an ordered sequence of nucleotide bases having a corresponding ordered sequence of base quality scores, and generate a compact text representation of the genetic sequencing read, the compact text representation including (1) a text string representing the ordered sequence of nucleotide bases and (2) a base quality text field indicative of the longest sub-sequence of the ordered sequence of nucleotide bases for which base quality scores satisfy a base quality score threshold, wherein the electronic data processing device identifies the longest sub-sequence of the ordered sequence of nucleotide bases.

14. The non-transitory storage medium of claim **13**, wherein:
the text string representing the ordered sequence of nucleotide bases comprises a sequence of letters comporting with a nucleotide base representation code in which:
the letter "A" or "a" represents an adenine nucleotide base,
the letter "C" or "c" represents a cytosine nucleotide base,
the letter "G" or "g" represents a guanine nucleotide base,
the letter "T" or "t" represents a thymine nucleotide base, and
the letter "U" or "u" represents a uracil nucleotide base; and
the compact text representation of the genetic sequencing read does not include a text string representing an ordered sequence of base quality scores.

15. The non-transitory storage medium of any one of claims **13-14**, wherein the stored instructions are further executable by an electronic data processing device **(30)** to reconstruct a genetic sequence by aligning genetic sequencing reads including said genetic sequencing, wherein the aligning includes defining an offset boundary for said genetic sequencing read based on the content of the base quality text field.

## Patentansprüche

1. Vorrichtung, umfassend:
eine elektronische Datenverarbeitungseinrichtung (30), die so konfiguriert ist, dass sie eine Kompakttextdarstellung eines genetischen Sequenzierungs-Reads generiert, wobei der genetische Sequenzierungs-Read eine geordnete Sequenz von Nucleotidbasen mit einer entsprechenden geordneten Sequenz von Basenqualitäts-Scores umfasst, wobei die Kompakttextdarstellung (1) eine die geordnete Sequenz von Nucleotidbasen darstellende Textfolge sowie (2) ein Basenqualitätstextfeld enthält, das für die längste Subsequenz der geordneten Sequenz von Nucleotidbasen indikativ ist, bei denen die entsprechenden Basenqualitäts-Scores einem Basenqualitäts-Score-Schwellenwert entsprechen, wobei die elektronische Datenverarbeitungseinrichtung die längste Subsequenz der geordneten Sequenz von Nucleotidbasen identifiziert; sowie
einen Raw-Reads-Speicher (22), der so konfiguriert ist, dass er die Kompakttextdarstellung speichert.

2. Vorrichtung nach Anspruch 1, wobei die Kompakttextdarstellung des genetischen Sequenzierungs-Reads enthält:
(2.1) ein erstes Basenqualitätstextfeld, das für die längste Subsequenz der geordneten Sequenz von Nucleotidbasen indikativ ist, bei denen die entsprechenden Basenqualitäts-Scores einem ersten Basenqualitäts-Score-Schwellenwert entsprechen, sowie
(2.2) ein zweites Basenqualitätstextfeld, das für die längste Subsequenz der geordneten Sequenz von Nucleotidbasen indikativ ist, bei denen die entsprechenden Basenqualitäts-Scores einem zweiten Basenqualitäts-Score-Schwellenwert entsprechen, der höher als der erste Basenqualitäts-Score-Schwellenwert ist.

3. Vorrichtung nach einem der Ansprüche 1-2, wobei die Kompakttextdarstellung des genetischen Sequenzierungs-Reads keine die geordnete Sequenz von Basenqualitäts-Scores darstellende Textfolge enthält.

4. Vorrichtung nach einem der Ansprüche 1-3, wobei das Basenqualitätstextfeld die längste Subsequenz durch Spezifizieren von einer der folgenden anzeigt:
der Position der ersten Nucleotidbase der Subsequenz in der geordneten Sequenz von Nucleotidbasen und der Position der letzten Nucleotidbase der Subsequenz in der geordneten Sequenz von Nucleotidbasen; sowie
der Position der ersten Nucleotidbase der Subsequenz in der geordneten Sequenz von Nucleotidbasen und der Anzahl von Nucleotidbasen in der Subsequenz.

5. Vorrichtung nach einem der Ansprüche 1-4, wobei das Basenqualitätstextfeld weiterhin den Basenqualitäts-Score-Schwellenwert anzeigt.

6. Vorrichtung nach einem der Ansprüche 1-5, wobei die elektronische Datenverarbeitungseinrichtung (30) weiterhin so konfiguriert ist, dass sie genetische Sequenzierungs-Reads, die besagten genetischen Sequenzierungs-Read enthalten, ausrichtet, um eine genetische Sequenz zu rekonstruieren, wobei der Ausrichtungsprozess das Definieren einer Versatzgrenze für den genetischen Sequenzierungs-Read, basierend auf dem Inhalt des Basenqualitätstextfeldes, umfasst.

7. Vorrichtung nach Anspruch 6, wobei die Versatzgrenze als eine Grenze der in dem Basenqualitätstextfeld identifizierten, längsten Subsequenz identifiziert wird.

8. Vorrichtung nach einem der Ansprüche 1-7, wobei die elektronische Datenverarbeitungseinrichtung (30) weiterhin so konfiguriert ist, dass sie die Kompakttextdarstellung des genetischen Sequenzierungs-Reads erweitert, um eine erweiterte Textdarstellung des genetischen Sequenzierungs-Reads zu generieren, die enthält: (1') eine Textfolge, welche die geordnete Sequenz von Nucleotidbasen darstellt, sowie (2') eine geordnete Sequenz von rekonstituierten Basenqualitäts-Scores entsprechend der geordneten Sequenz von Nucleotidbasen;
wobei die rekonstituierten Basenqualitäts-Scores größer als der Basenqualitätsschwellenwert für Basen der durch das Basenqualitätstextfeld identifizierten längsten Subsequenz sind.

9. Verfahren zur Anwendung bei Verarbeiten eines genetischen Sequenzierungs-Reads mit einer Basensequenz, die durch Verarbeiten einer Gewebeprobe (10) erfasst wird, wobei gemäß dem Verfahren:
eine Kompakttextdarstellung des genetischen Sequenzierungs-Reads generiert wird, wobei der genetische Sequenzierungs-Read eine geordnete Sequenz von Nucleotidbasen mit einer entsprechenden geordneten Sequenz von Basenqualitäts-Scores umfasst, wobei die Kompakttextdarstellung (1) eine die Basensequenz darstellende Textfolge sowie (2) ein Basenqualitätstextfeld enthält, das für die längste Subsequenz der Basensequenz indikativ ist, bei denen Basenqualitäts-Scores der Basen der Subsequenz einem Basenqualitäts-Score-Schwellenwert entsprechen, und
die Kompakttextdarstellung des genetischen Sequenzierungs-Reads in einem Raw-Reads-Speicher (22) gespeichert wird;
wobei das Generieren durch eine elektronische Datenverarbeitungseinrichtung (30) durchgeführt wird, und wobei die elektronische Datenverarbeitungseinrichtung die längste Subsequenz der geordneten Sequenz von Nucleotidbasen identifiziert.

10. Verfahren nach Anspruch 9, wobei:
die Kompakttextdarstellung des genetischen Sequenzierungs-Reads keine Textfolge enthält, die eine Sequenz von Basenqualitäts-Scores entsprechend der Basensequenz darstellt; und
die Textfolge die Basensequenz mit einer Sequenz von Buchstaben darstellt, die mit einem Basendarstellungscode übereinstimmen, in dem der Buchstabe "A" oder "a" eine Adenin-Base darstellt, der Buchstabe "C" oder "c" eine Cytosin-Base darstellt, der Buchstabe "G" oder "g" eine Guanin-Base darstellt, der Buchstabe "T" oder "t" eine Thymin-Base darstellt und der Buchstabe "U" oder "u" eine Uracil-Base darstellt.

11. Verfahren nach einem der Ansprüche 9-10, wobei:
das Basenqualitätstextfeld die längste Subsequenz durch Spezifizieren von einer der folgenden anzeigt:
(i) der ersten Basenposition und der letzten Basenposition der Subsequenz sowie
(ii) der ersten Basenposition und der Anzahl von Basen in der Subsequenz; und
das Basenqualitätstextfeld weiterhin den Basenqualitäts-Score-Schwellenwert identifiziert.

12. Verfahren nach einem der Ansprüche 9-11, wonach weiterhin:
genetische Sequenzierungs-Reads, die besagten genetischen Sequenzierungs-Read enthalten, ausgerichtet werden, um eine genetische Sequenz zu rekonstruieren, wobei die Ausrichtung das Definieren einer Versatzgrenze für den genetischen Sequenzierungs-Read als eine Grenze der in dem Basenqualitätstextfeld identifizierten, längsten Subsequenz umfasst;
wobei das Ausrichten durch eine elektronische Datenverarbeitungseinrichtung (30) durchgeführt wird.

13. Nicht-transitorisches Speichermedium zur Speicherung von, durch eine elektronische Datenverarbeitungseinrichtung (30) ausführbaren Instruktionen zur Verarbeitung eines genetischen Sequenzierungs-Reads, wobei der genetische Sequenzierungs-Read eine geordnete Sequenz von Nucleotidbasen mit einer entsprechenden geordneten Sequenz von Basenqualitäts-Scores umfasst, sowie zur Generierung einer Kompakttextdarstellung des genetischen Sequenzierungs-Reads, wobei die Kompakttextdarstellung (1) eine die geordnete Sequenz von Nucleotidbasen darstellende Textfolge sowie (2) ein Basenqualitätstextfeld enthält, das für die längste Subsequenz der geordneten Sequenz von Nucleotidbasen indikativ ist, bei denen Basenqualitäts-Scores einem Basenqualitäts-Score-Schwellenwert entsprechen, wobei die elektronische Datenverarbeitungseinrichtung die längste Subsequenz der geordneten Sequenz von Nucleotidbasen identifiziert.

14. Nicht-transitorisches Speichermedium nach Anspruch 13, wobei:
die Textfolge, welche die geordnete Sequenz von Nucleotidbasen darstellt, eine Sequenz von Buchstaben umfasst, die mit einem Basendarstellungscode übereinstimmen, in dem:
der Buchstabe "A" oder "a" eine Adenin-Nucleotidbase darstellt,
der Buchstabe "C" oder "c" eine Cytosin-Nucleotidbase darstellt,
der Buchstabe "G" oder "g" eine Guanin-Nucleotidbase darstellt,
der Buchstabe "T" oder "t" eine Thymin-Nucleotidbase darstellt und
der Buchstabe "U" oder "u" eine Uracil-Nucleotidbase darstellt; und
die Kompakttextdarstellung des genetischen Sequenzierungs-Reads keine, eine geordnete Sequenz von Basenqualitäts-Scores darstellende Textfolge enthält.

15. Nicht-transitorisches Speichermedium nach den Ansprüchen 13-14, wobei die gespeicherten Instruktionen weiterhin durch eine elektronische Datenverarbeitungseinrichtung (30) ausführbar sind, um eine genetische Sequenz durch Ausrichten genetischer Sequenzierungs-Reads, die gesagten genetischen Sequenzierungs-Read enthalten, zu rekonstruieren, wobei die Ausrichtung das Definieren einer Versatzgrenze für den genetischen Sequenzierungs-Read, auf dem Inhalt des Basenqualitätstextfeldes basierend, umfasst.

## Revendications

1. Appareil comprenant :
un dispositif électronique de traitement de données (30) conçu pour générer une représentation textuelle compacte d'une lecture de séquençage génétique, la lecture de séquençage génétique comprenant une séquence ordonnée de bases nucléotidiques ayant une séquence ordonnée correspondante de scores de qualité de base, la représentation textuelle compacte incluant (1) une chaîne de texte représentant la séquence ordonnée de bases nucléotidiques et (2) un champ textuel de qualité de base indiquant la plus longue sous-séquence de la séquence ordonnée de bases nucléotidiques pour laquelle les scores de qualité de base correspondants satisfont un seuil de score de qualité de base, dans lequel le dispositif électronique de traitement de données identifie la plus longue sous-séquence de la séquence ordonnée de bases nucléotidiques ; et
une mémoire de lectures brutes (22) conçue pour stocker la représentation textuelle compacte.

2. Appareil selon la revendication 1, dans lequel la représentation textuelle compacte de la lecture de séquençage génétique inclut :
(2.1) un premier champ textuel de qualité de base indiquant la plus longue sous-séquence de la séquence ordonnée de bases nucléotidiques pour laquelle les scores de qualité de base correspondants satisfont un premier seuil de score de qualité de base, et
(2.2) un second champ textuel de qualité de base indiquant la plus longue sous-séquence de la séquence ordonnée de bases nucléotidiques pour laquelle les scores de qualité de base correspondants satisfont un second seuil de score de qualité de base qui est supérieur au premier seuil de score de qualité de base.

3. Appareil selon l'une quelconque des revendications 1 et 2, dans lequel la représentation textuelle compacte de la lecture de séquençage génétique n'inclut pas de chaîne de texte représentant la séquence ordonnée de scores de qualité de base.

4. Appareil selon l'une quelconque des revendications 1 à 3, dans lequel le champ textuel de qualité de base indique la plus longue sous-séquence en spécifiant soit :
la position de la première base nucléotidique de la sous-séquence dans la séquence ordonnée de bases nucléotidiques et la position de la dernière base nucléotidique de la sous-séquence dans la séquence ordonnée de bases nucléotidiques ; soit
la position de la première base nucléotidique de la sous-séquence dans la séquence ordonnée de bases nucléotidiques et le nombre de bases nucléotidiques dans la sous-séquence.

5. Appareil selon l'une quelconque des revendications 1 à 4, dans lequel le champ textuel de qualité de base indique en outre le seuil de score de qualité de base.

6. Appareil selon l'une quelconque des revendications 1 à 5, dans lequel le dispositif électronique de traitement de données (30) est conçu en outre pour aligner des lectures de séquençage génétique incluant ladite lecture de séquençage génétique pour reconstituer une séquence génétique, dans lequel le processus d'alignement inclut la définition d'une frontière décalée pour ladite lecture de séquençage génétique basée sur le contenu du champ textuel de qualité de base.

7. Appareil selon la revendication 6, dans lequel la frontière décalée est définie comme étant une frontière de la plus longue sous-séquence identifiée dans le champ textuel de qualité de base.

8. Appareil selon l'une quelconque des revendications 1 à 7, dans lequel le dispositif électronique de traitement de données (30) est conçu en outre pour développer la représentation textuelle compacte de la lecture de séquençage génétique pour générer une représentation textuelle développée de la lecture de séquençage génétique incluant (1') une chaîne de texte représentant la séquence ordonnée de bases nucléotidiques et (2') une séquence ordonnée de scores de qualité de base reconstitués correspondant à la séquence ordonnée de bases nucléotidiques ;
dans lequel les scores de qualité de base reconstitués sont supérieurs au seuil de qualité de base pour les bases de la plus longue sous-séquence identifiée par le champ textuel de qualité de base.

9. Procédé opérant sur une lecture de séquençage génétique comprenant une séquence de bases acquise par traitement d'un échantillon de tissu (10), le procédé comprenant :
la génération d'une représentation textuelle compacte de la lecture de séquençage génétique, la lecture de séquençage génétique comprenant une séquence ordonnée de bases nucléotidiques ayant une séquence ordonnée correspondante de scores de qualité de base, la représentation textuelle compacte incluant (1) une chaîne de texte représentant la séquence de bases et (2) un champ textuel de qualité de base indiquant la plus longue sous-séquence de la séquence de bases pour laquelle les scores de qualité de base de la sous-séquence satisfont un seuil de score de qualité de base ; et
le stockage de la représentation textuelle compacte de la lecture de séquençage génétique dans une mémoire de lectures brutes(22) ;
dans lequel la génération est réalisée par un dispositif électronique de traitement de données (30), et dans lequel le dispositif électronique de traitement de données identifie la plus longue sous-séquence de la séquence ordonnée de bases nucléotidiques.

10. Procédé selon la revendication 9, dans lequel :
la représentation textuelle compacte de la lecture de séquençage génétique n'inclut pas de chaîne de texte représentant une séquence de scores de qualité de base correspondant à la séquence de bases ; et
la chaîne de texte représentant la séquence de bases comprend une séquence de lettres comportant un code de représentation de base dans lequel la lettre « A » ou « a » représente une base adénine, la lettre « C » ou « c » représente une base cytosine, la lettre « G » ou « g » représente une base guanine, la lettre « T » ou « t » représente une base thymine, et la lettre « U » ou « u » représente une base uracile.

11. Procédé selon l'une quelconque des revendications 9 ou 10, dans lequel :
le champ textuel de qualité de base indique la plus longue sous-séquence en spécifiant soit : (i) la première position de base et la dernière position de base de la sous-séquence soit (ii) la première position de base et le nombre de bases dans la sous-séquence; et
le champ textuel de qualité de base identifie en outre le seuil de score de qualité de base.

12. Procédé selon l'une quelconque des revendications 9 à 11, comprenant en outre :
l'alignement de lectures de séquençage génétique incluant ladite lecture de séquençage génétique pour reconstituer une séquence génétique dans lequel l'alignement inclut la définition d'une frontière décalée pour ladite lecture de séquençage génétique comme étant frontière de la plus longue sous-séquence identifiée dans le champ textuel de qualité de base ;
dans lequel l'alignement est effectué par un dispositif électronique de traitement de données (30).

13. Support de stockage non transitoire stockant des instructions exécutables par un dispositif électronique de traitement de données (30) pour traiter une lecture de séquençage génétique, la lecture de séquençage génétique comprenant une séquence ordonnée de bases nucléotidiques ayant une séquence ordonnée correspondante de scores de qualité de base, et pour générer une représentation textuelle compacte de la lecture de séquençage génétique, la représentation textuelle compacte incluant (1) une chaîne de texte représentant la séquence ordonnée de bases nucléotidiques et (2) un champ textuel de qualité de base indiquant la plus longue sous-séquence de la séquence ordonnée de bases nucléotidiques pour laquelle des scores de qualité de base satisfont un seuil de score de qualité de base, dans lequel le dispositif électronique de traitement de données identifie la plus longue sous-séquence de la séquence ordonnée de bases nucléotidiques.

14. Support de stockage non transitoire selon la revendication 13, dans lequel :
la chaîne de texte représentant la séquence ordonnée de bases nucléotidiques comprend une séquence de lettres comportant un code de représentation de base nucléotidique dans lequel :
la lettre « A » ou « a » représente une base nucléotidique adénine,
la lettre « C » ou « c » représente une base nucléotidique cytosine,
la lettre « G » ou « g » représente une base nucléotidique guanine,
la lettre « T » ou « t » représente une base nucléotidique thymine, et
la lettre « U » ou « u » représente une base nucléotidique uracile ; et
la représentation textuelle compacte de la lecture de séquençage génétique n'inclut pas de chaîne de texte représentant une séquence ordonnée de scores de qualité de base.

15. Support de stockage non transitoire selon l'une quelconque des revendications 13 et 14, dans lequel les instructions stockées sont exécutables en outre par un dispositif électronique de traitement de données (30) pour reconstituer une séquence génétique en alignant des lectures de séquençage génétique incluant ledit séquençage génétique, dans lequel l'alignement inclut la définition d'une frontière décalée pour ladite lecture de séquençage génétique basée sur le contenu du champ textuel de qualité de base.
